# EUROPEAN PATENT SPECIFICATION

(11) **EP 1 517 994 B1**
(45) Date of publication and mention of the grant of the patent: **24.01.2007**
(21) Application number: 03729905.4
(22) Date of filing: 19.06.2003
(51) Int. Cl.: C12P 19/26, C08B 37/00

(54) **FLOCCULATION WITH DIVALENT SALT**
AUSFLOCKUNG MIT ZWEIWERTIGEM SALZ
FLOCULATION AU MOYEN D'UN SEL DIVALENT

(30) Priority: 20.06.2002 DK 200200944
(43) Date of publication of application: 30.03.2005
(73) Proprietor: Novozymes Biopolymer A/S, 2880 Bagsvaerd (DK)
(72) Inventor: THWAITES, Eric, DK-2300 Copenhagen (DK)
(74) Representative: Kofoed, Gertrud Sonne
(86) International application number: PCT/DK2003/000409
(87) International publication number: WO 2004/001054

(56) References cited:
- EP-A- 0 038 265
- WO-A-91/03559
- US-A- 3 711 462
- US-A- 4 094 739
- US-A- 4 517 295
- US-A- 4 780 414
- US-A- 4 782 046
- US-A- 5 316 926
- BAJPAI P ET AL: "CLARIFICATION OF BACTERIAL BROTH CONTAINING HIGH ALPHA AMYLASE ACTIVITY" BIOTECHNOLOGY TECHNIQUES, vol. 4, no. 4, 1990, pages 227-232, XP008008107 ISSN: 0951-208X

## Description

### TECHNICAL FIELD

The present invention relates to a simple and elective method for flocculation of a Bacillus cell producing a glycosaminoglucan of interest, such as hyaluronic acid from a fermentation broth.

### BACKGROUND ART

Hyaluronic acid (HA) is a mucoid polysaccharide of biological origin, which is widely distributed in nature. For example, it is known that hyaluronic acid is present in animal tissues such as umbilical cord, synovial fluid, vitreous humor, rooster comb, and various connective tissues such as skin and cartilage.

Hyaluronic acid, at high molecular weight, is viscous and able to maintain a jelly state, acting as a lubricant, preventing the invasion of bacteria and retaining water.

Due to the above mentioned properties, hyaluronic acid has technical and cosmetic uses, e.g., hyaluronic acid is able to retain the tonicity and elasticity of the skin, Pharmaceutical use of hyaluronic acid is widely described in the literature, e.g., as an eye vitreous or as a supportive medium in ophthalmic surgery or as a joint fluid replacement.

Thanks to its highly hydrophilic nature, hyaluronic acid may also be used in cosmetic products such as lotions and creams.

Hyaluronic acid may be extracted and purified from e.g. umbilical cords, from rooster combs or from group A and C Streptococci.

The hyaluronic acid may also be produced by recombinant production in a host cell such as a bacterial cell or a fungal cell.

When hyaluronic acid is produced by fermentation of a micro-organism, the cell mass is conventionally removed by filtration or centrifugation at high dilution. Flocculation allows a more efficient cell removal and also the use of a wider range of cell removal techniques (e.g. drum filtration, dead end filtration, centrifugation etc). However, flocculation chemicals can adversely affect the product or impede down stream processing strategies.

### SUMMARY OF THE INVENTION

We have found that it is possible to flocculate a Bacillus cell producing a glycosaminoglucan of interest, in a very efficient way: Addition of a divalent salt has been shown to create small floes which can be easily separated from the cell broth whilst not reducing product yield, or impeding subsequent down stream operations.

The present invention provides a method of flocculating a Bacillus cell and/or removing high molecular weight contaminants from a fermentation broth, comprising adding a divalent salt to the fermentation broth comprising a glycosaminoglucan of interest after which the Bacillus cell and/or the high molecular weight contaminants are removed, wherein said Bacillus cell produces the glycosaminoglucan of interest.

### DETAILED DISCLOSURE OF THE INVENTION

The present invention provides a method for flocculating a Bacillus cell, producing a glycosaminoglucan of interest, from a fermentation broth comprising adding a divalent salt to the fermentation broth whereafter the Bacillus cell is removed.

### Glycosaminoglucans

According to the invention a glycosaminoglucan may be any carbohydrate polymer having a molecular weight of at least 10,000 Daltons; preferably a molecular weight of at least 20,000 Daltons; more preferably a molecular weight of at least 30,000 Daltons.

Preferred glycosaminoglycans are hyaluronic acid, chondroitin sulphate, heparin, heparin sulphate, dermatan sulphate, and keratin sulphate. Hyaluronic acid is constituted by alternating and repeating units of D-glucoronic acid and N-acetyl-D-glucosamine, to form a linear chain having a molecular weight of up to 15,000,000 Daltons.

Preferred glycosaminoglucans according to the invention are glycosaminoglucans having a molecular weight of from 10,000 Daltons to 15,000,000 Daltons.

It is to be noted that the term "hyaluronic acid" in the present application and claims may mean indifferently hyaluronic acid in its acidic form or in its salt form such as for example sodium hyaluronate, potassium hyaluronate, magnesium hyaluronate, calcium hyaluronate, or others.

### Fermentation broth

The glycosaminoglucan may be obtained from any fermentation broth. The glycosaminoglucan may furthermore be one which is producible by a method comprising cultivating a host cell.

The host cell is a *Bacillus cell, e.g., Bacillus alkalophilus, Bacillus amyloliguefaciens, Bacillus brevis, Bacillus circulans, Bacillus clausii, Bacillus* coagulans, *Bacillus firmus, Bacillus lautus, Bacillus lentus, Bacillus licheniformis, Bacillus megaterium, Bacillus pumilus, Bacillus stearothermophilus, Bacillus subtilis, or Bacillus thuringiensis.* In a preferred embodiment, the Bacillus host cell is a *Bacillus lentus* cell, a *Bacillus licheniformis* cell, a *Bacillus stearothermophilus* cell or a *Bacillus subtilis* cell. Mutant *Bacillus subtilis* cells particularly adapted for recombinant expression are described in WO 98/22898.

The Bacillus cell producing the glycosaminoglucan of interest is cultivated in a nutrient medium suitable for production of the glycosaminoglucan using methods known in the art. For example, the Bacillus cell may be cultivated by shake flask cultivation, small-scale or large-scale fermentation (including but not limited to continuous, batch, fed-batch, or solid state fermentations) in laboratory or industrial fermentors. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection).

### High molecular weight contaminants / impurities

Non-glycosaminoglucan materials in the same or higher molecular weight range as the glycosaminoglucan of interest itself, such as, but not limited to: levan, glutamic adds, nudeic acids, cellular debris and components etc.

By using the method of the invention it is possible to remove high molecular weight contaminants/impurities; it is also possible to remove these impurities from cell free preparations of the glycosaminoglucan of interest.

### Flocculation

The method of the invention may be applied to an untreated fermentation broth or to a fermentation broth that has first been subjected to, but not limited to, e.g., a pH adjustment, a temperature adjustment, and/or a water dilution.

According to the invention it has been found that an addition of a divalent saft to the fermentation broth results in a flocculation with small flocs which can be easily separated from the cell broth.

The evaluation of floc sizes is normally done by comparing the observed flocs with a photograph of various floc sizes.

It will often be an advantage to dilute the fermentation broth with water before, simultaneously or after the addition of the divalent salt. Depending on the yield of the glycosaminoglucan and the wanted viscosity a dilution with 100-500% (w/w) of water of the fermentation broth will be appropriate; preferably a dilution with 100-400% (w/w) of water of the fermentation broth.

It will often be an advantage to heat the fermentation broth before, simultaneously or after addition of the divalent salt, or to heat the fermentation broth before, simultaneously or after the dilution depending on the yield of the glucosaminoglucan of interest and the wanted viscosity. The fermentation broth is preferably heated to a temperature above 10°C, in particular to a temperature between 30°C and 60°C.

It will often be an advantage to pH adjust the fermentation broth before, simultaneously or after addition of the divalent salt and or the dilution. Preferably the fermentation broth is adjusted to a pH between 6.5 and 14, in particular to a pH between 7.5 and 8.5.

### Divalent salts

According to the invention a surprisingly good flocculating agent is a divalent salt, in particular a calcium and/or a magnesium salt. A preferred divalent salt is a phosphate, a sulphate or a chloride, e.g., calcium chloride. A preferred embodiment is a calcium salt.

The minimum quantity of a divalent salt which will flocculate is dependent upon the micro-organism of the fermentation broth, the constituents of the fermentation broth and the type and the concentration of the salt itself. It should also be noted that overdosing of the divalent salt may reduce the yield. The divalent salt should be added to at least 0.1 g of divalent salt per gram of dry mass of the micro-organism of the fermentation broth, preferably the divalent salt may be added in a range of 0.5-25 g of divalent salt per gram of dry mass of the micro-organism of the fermentation broth, more preferably the divalent salt may be added in a range of 0.5-15 g of divalent salt per gram of dry mass of the micro-organism of the fermentation broth, most preferably the divalent salt may be added in a range of 0.5-8 g of divalent salt per gram of dry mass of the micro-organism of the fermentation broth, in particular the divalent salt may be added in a range of 0.5-3.5 g of divalent salt per gram of dry mass of the micro-organism of the fermentation broth.

The dosage of the divalent salt is typically done either in-line or in a mixing tank or by any other method known in the art.

It may be an advantage, in addition to the divalent salt, to add one or more flocculating agents such as an aluminate, e.g., NaAlO2, or a cationic or an anionic polymer.

After addition of the divalent salt, and optionally the other flocculating agents, the micro-organisms are removed by methods known in the art such as, but not limited to, filtration, e.g. drum filtration, membrane filtration, filterpress dead end filtration, cross-flow filtration, or centrifugation.

### Subsequent downstream operations

The resulting glycosaminoglucan may be further isolated by methods known in the art. For example, the glycosaminoglucan may be recovered by conventional procedures including, but not limited to, further filtration, extraction, spray-drying, evaporation, precipitation or crystallization. The isolated glycosaminoglucan may then be further purified and/or modified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), or extraction.

The invention is further illustrated in the following examples which are not intended to be in any way limiting to the scope of the invention as claimed.

### EXAMPLE 1

### HA Flocculation Example

Source: GMM *B. Subtilis* containing the hyaluronic acid cassette produced as described in copending US application: US 60/342644.

### Fermentation:

We used a 1500 litre scale; used a defined medium employing inorganic nitrogen as the sole nitrogen source and sucrose dosing as the carbon source.

Final dry cell mass in the fermentation broth was approximately 15 to 20 gl⁻¹ on a dry cell weight basis. (Difficult to assess precisely due to high viscosity of the broth).

Hyaluronic acid in the fermentation broth was approximately 10 gl⁻¹ with an average molecular weight of approximately 1,400,000 Da. (Measured by ELISA and GPC-MALLS respectively).

### Cell Separation:

Dilution: 200% (1 in 3) dilution with water

Temperature: 10°C

pH 7.0

Flocculation: 2 wt % Calcium chloride relative to the culture broth, or approximately 1.0 to 1.3 g of Calcium chloride per g of dry cells.

### Cell removal:

The flocculated cells were easily removed from the hyaluronic acid containing broth under normal equipment operating conditions and fluxes with little loss of yield by one or more of the following methods:
a: Dead end "nutch " filtration. Normal operating conditions.
b: Centrifugation at bench scale in 50 ml centrifuge tubes. Normal operating conditions.
c: Drum filtration (2.5 m² area). Normal operating conditions.
d: Centrifugation at pilot scale. Normal operating conditions.

We tested (a)-(d), and in each case we obtained a remarkably clear, straw coloured liquid containing the majority of the hyaluronic acid in the original fermentation broth.

The ease of separation would suggest that cell separation would also be improved on other cell separation equipment (e.g. filter press, microfiltration, etc).

### EXAMPLE 2

### HA Flocculation Example pilot scale, pH 6.5

Source: GMM *B. Subtilis* containing the hyaluronic acid cassette produced as described in copending US application: US 60/342644.

### Fermentation:

We used a 1500 litre scale; used a defined medium employing inorganic nitrogen as the sole nitrogen source and sucrose dosing as the carbon source.

Final dry cell mass in the fermentation broth was approximately 10 to 15 gl⁻¹ on a dry cell weight basis. (Difficult to assess precisely due to high viscosity of the broth).

Hyaluronic acid in the fermentation broth was approximately 12 gl⁻¹ with an average molecular weight of approximately 1,000,000 Da. (Measured by ELISA and GPC-MALLS respectively).

### Cell Separation:

Dilution: 300% (1 in 4) dilution with water

Temperature 35 °C

Flocculation: 2 wt % Calcium chloride relative to the culture broth or 1.3 to 2.0 g of Calcium chloride per g of dry cells.

pH 6.5

### Cell removal:

The flocculated cells were easily removed from the hyaluronic acid containing broth under normal equipment operating conditions and fluxes with little loss of yield by one or more of the following methods:
a: Dead end "nutch " filtration. Normal operating conditions.
b: Centrifugation at bench scale in 50 ml centrifuge tubes. Normal operating conditions.
c: Drum filtration (2.5 m² area). Normal operating conditions.
d: Centrifugation at pilot scale. Normal operating conditions.

We tested (a)-(d), and in each case we obtained a remarkably clear, yellow straw coloured liquid containing the majority of the hyaluronic acid in the original fermentation broth.

The ease of separation would suggest that cell separation would also be improved on other cell separation equipment (e.g. filter press, microfiltration, etc).

### EXAMPLE 3

### HA Flocculation Example process scale, pH 8

Source: GMM *B*. *Subtilis* containing the hyaluronic acid cassette produced as described in copending US application: US 60/342644.

### Fermentation:

We used a 20 000 litre scale; used a defined medium employing inorganic nitrogen as the sole nitrogen source and sucrose dosing as the carbon source.

Final dry cell mass in the fermentation broth was approximately 8 to 10 gl⁻¹ on a dry cell weight basis.

Hyaluronic acid in the fermentation broth was approximately 10 gl⁻¹ with an average molecular weight of approximately 1,000,000 Da. (Measured by ELISA and GPC-MALLS respectively).

### Cell Separation:

Dilution: 400% (1 in 5) dilution with water

Temperature 45°C

pH 8.0

Flocculation: 2 wt % Calcium chloride relative to the culture broth or approximately 2.0 to 2.5 g of Calcium chloride per g of dry cells.

### Cell removal:

The flocculated cells were easily removed from the hyaluronic acid containing broth under normal equipment operating conditions and fluxes with little loss of yield by one or more of the following methods:
a: Dead end "nutch" filtration. Normal operating conditions.
b: Centrifugation at bench scale in 50 ml centrifuge tubes. Normal operating conditions.
c: Drum filtration (process scale). Normal operating conditions.
d: Filter press (pilot scale). Normal operating conditions.

We tested (a)-(d), and in each case we obtained a remarkably clear, straw coloured liquid containing the majority of the hyaluronic acid in the original fermentation broth. High molecular weight contaminants (as assessed by GPC analysis before and after treatment as well as by enzymatic digestion) were comprehensively removed.

The ease of separation would suggest that cell separation would also be improved on other cell separation equipment (e.g. microfiltration, etc). Operation at this dilution and temperature reduced viscosity such that flux rates were higher than in examples 1 or 2. Further, the cellular and other contaminant clearance (measured by cell counts and turbidity) were also greater than in examples 1 or 2.

### EXAMPLE 4

### HA Flocculation Example pilot scale, contaminant removal experiment

Source: GMM *B. Subtilis* containing the hyaluronic acid cassette produced as described in copending US application: US 60/342644.

### Fermentation:

We used a 1500 litre scale; used a defined medium employing inorganic nitrogen as the sole nitrogen source and sucrose dosing as the carbon source.

Final dry cell mass in the fermentation broth was approximately 8 to 10 gl⁻¹ on a dry cell weight basis.

Hyaluronic acid in the fermentation broth was approximately 10 gl⁻¹ with an average molecular weight of approximately 1,000,000 Da. (Measured by ELISA and GPC-MALLS respectively).

### Cell Separation:

Dilution: 300% (1 in 4) dilution with water

Temperature 15 to 60°C

pH 5.0 to 10.0

Flocculation: 2 wt % Calcium chloride relative to the culture broth or approximately 2.0 to 2.5 g of Calcium chloride per g of dry cells.

### Cell removal:

The flocculated cells were easily removed from the hyaluronic acid containing broth under normal equipment operating conditions and fluxes with little loss of yield by a dead end "nutch " filtration. Normal operating conditions. A control without calcium chloride failed to filter or to clarify the treated culture broth.

Investigation of the collected filtrates by GPC-MALLS, enzymatic digestion and other analyses revealed that the degree of high molecular weight contaminant removal could be controlled by pH with the highest degree of removal being below pH 5.5 and at pH > 7.5; pH 8 being the preferred value for process operations. Degree of high molecular weight contaminant removal was found to be independent of temperature or dilution.

## Claims

1. A method of flocculating a *Bacillus* cell and/or removal of high molecular weight contaminants from a fermentation broth, comprising adding a divalent salt to the fermentation broth comprising a glycosaminoglucan of interest after which the *Bacillus* cell and/or the high molecular weight contaminants are removed, wherein said *Bacillus* cell produces the glycosaminoglucan of interest.

2. The method according to claim 1, wherein the glycosaminoglycan is hyaluronic acid.

3. The method according to claim 1, wherein the glycosaminoglycan has a molecular weight of from 10,000 Daltons to 15,000,000 Daltons.

4. The method according to claim 1, wherein the divalent salt is a calcium salt and/or a magnesium salt.

5. The method according to claim 1, wherein the divalent salt is added at a concentration of 0.1-25 g of divalent salt per gram of dry mass of micro-organism.

6. The method according to claim 1, wherein the micro-organism is removed by filtration.

7. The method according to claim 1, wherein the fermentation broth is diluted with water before, simultaneously or after the addition of the divalent salt.

8. The method according to claim 7, wherein the fermentation broth is diluted with 100-500% (w/w) water.

9. The method according to claim 8, wherein the fermentation broth is diluted with 100-400% (w/w) water.

10. The method according to claim 1, wherein the fermentation broth is adjusted to a pH between 6.5 and 14.

11. The method according to claim 10, wherein the fermentation broth is adjusted to a pH between 7.5 and 8.5.

12. The method according to claim 1, wherein the fermentation broth is adjusted to a pH below 5.5 after which the high molecular contaminants are removed.

13. The method according to claim 1, wherein the fermentation broth is heated to a temperature between 30°C and 60°C.

14. The method according to claim 1, wherein additionally one or more other flocculating agents are added to the fermentation broth.

## Patentansprüche

1. Verfahren zum Ausflocken einer *Bacillus*-Zelle und/oder zum Entfernen von Kontaminanten hohen Molekulargewichts aus einer Fermentationsbrühe, umfassend das Zufügen eines bivalenten Salzes zu der Fermentationsbrühe, die ein interessierendes Glycosaminoglucan umfasst, wonach die *Bacillus*-Zelle und/oder die Kontaminanten hohen Molekulargewichts entfernt werden, wobei die *Bacillus-*Zelle das interessierende Glycosaminoglucan herstellt.

2. Verfahren nach Anspruch 1, wobei das Glycosaminoglucan Hyaluronsäure ist.

3. Verfahren nach Anspruch 1, wobei das Glycosaminoglucan ein Molekulargewicht von 10.000 Dalton bis 15.000.000 Dalton besitzt.

4. Verfahren nach Anspruch 1, wobei das bivalente Salz ein Kalziumsalz und/oder ein Magnesiumsalz ist.

5. Verfahren nach Anspruch 1, wobei das bivalente Salz in einer Konzentration von 0,1 bis 25 g des bivalenten Salzes pro Gramm Trockenmasse des Mikroorganismus zugefügt wird.

6. Verfahren nach Anspruch 1, wobei der Mikroorganismus durch Filtration entfernt wird.

7. Verfahren nach Anspruch 1, wobei die Fermentationsbrühe vor, gleichzeitig mit oder nach der Zugabe des bivalenten Salzes mit Wasser verdünnt wird.

8. Verfahren nach Anspruch 7, wobei die Fermentationsbrühe mit 100 bis 500 % (Gew./Gew.) Wasser verdünnt wird.

9. Verfahren nach Anspruch 8, wobei die Fermentationsbrühe mit 100 bis 400 % (Gew./Gew.) Wasser verdünnt wird.

10. Verfahren nach Anspruch 1, wobei die Fermentationsbrühe auf einen pH zwischen 6,5 und 14 eingestellt wird.

11. Verfahren nach Anspruch 10, wobei die Fermentationsbrühe auf einen pH zwischen 7,5 und 8,5 eingestellt wird.

12. Verfahren nach Anspruch 1, wobei die Fermentationsbrühe auf einen pH unter 5,5 eingestellt wird, wonach die Kontaminanten hohen Molekulargewichts entfernt werden.

13. Verfahren nach Anspruch 1, wobei die Fermentationsbrühe auf eine Temperatur zwischen 30 °C und 60 °C erhitzt wird.

14. Verfahren nach Anspruch 1, wobei zusätzlich ein oder mehrere andere Ausflockungsmittel zu der Fermentationsbrühe zugefügt werden.

## Revendications

1. Procéder pour floculer une cellule de bacille et/ou retirer des contaminants de haut poids moléculaire d'un bouillon de fermentation, comprenant l'ajout d'un sel divalent au bouillon de fermentation comprenant un glycosaminoglycane d'intérêt, puis la cellule de bacille et/ou les contaminants de haut poids moléculaire sont retirés, dans lequel ladite cellule de bacille produit le glycosaminoglycane d'intérêt.

2. Procédé selon la revendication 1, dans lequel le glycosaminoglycane est l'acide hyaluronique.

3. Procédé selon la revendication 1, dans lequel le glycosaminoglycane a un poids moléculaire de 10 000 Daltons à 15 000 000 Daltons.

4. Procédé selon la revendication 1, dans lequel le sel divalent est un sel de calcium et/ou un sel de magnésium.

5. Procédé selon la revendication 1, dans lequel le sel divalent est ajouté à une concentration de 0,1-25 g de sel divalent par gramme de masse sèche de microorganisme.

6. Procédé selon la revendication 1, dans lequel le microorganisme est retiré par filtration.

7. Procédé selon la revendication 1, dans lequel le bouillon de fermentation est dilué avec de l'eau avant, pendant ou après l'ajout du sel divalent.

8. Procédé selon la revendication 7, dans lequel le bouillon de fermentation est dilué avec 100-500 % (p/p) d'eau.

9. Procédé selon la revendication 8, dans lequel le bouillon de fermentation est dilué dans 100-400% (p/p) d'eau.

10. Procédé selon la revendication 1, dans lequel le bouillon de fermentation est ajusté à un pH entre 6,5 et 14.

11. Procédé selon la revendication 10, dans lequel le bouillon de fermentation est ajusté à un pH entre 7,5 et 8,5.

12. Procédé selon la revendication 1, dans lequel le bouillon de fermentation est ajusté à un pH inférieur à 5,5, puis les contaminants de haut poids moléculaire sont retirés.

13. Procédé selon la revendication 1, dans lequel le bouillon de fermentation est chauffé à une température entre 30 et 60°C.

14. Procédé selon la revendication 1, dans lequel en outre un ou plusieurs autres agents de floculation sont ajoutés au bouillon de fermentation.
